# EUROPÄISCHE PATENTSCHRIFT

(11) **EP 4 172 743 B1**
(45) Veröffentlichungstag und Bekanntmachung des Hinweises auf die Patenterteilung: **27.03.2024**
(21) Anmeldenummer: 22712820.4
(22) Anmeldetag: 25.02.2022
(51) Int. Cl.: G06F 3/01, A61L 9/12, A61L 9/14

(54) **VERFAHREN UND SYSTEM ZUR DARSTELLUNG EINER VIRTUELLEN REALITÄT**
METHOD AND SYSTEM FOR DISPLAYING A VIRTUAL REALITY
PROCÉDÉ ET SYSTÈME DE REPRÉSENTATION D'UNE RÉALITÉ VIRTUELLE

(30) Priorität: 25.02.2021 DE 102021104553; 18.06.2021 DE 102021115896; 04.01.2022 DE 102022100108
(43) Veröffentlichungstag der Anmeldung: 03.05.2023
(73) Patentinhaber: Dayholi GmbH, 8046 Graz (AT)
(72) Erfinder: HOLZLEITHNER, Fjolla, 8046 Graz (AT)
(74) Vertreter: Canzler & Bergmeier Patentanwälte Partnerschaft mbB
(86) Internationale Anmeldenummer: PCT/EP2022/054792
(87) Internationale Veröffentlichungsnummer: WO 2022/180210

(56) Entgegenhaltungen:
- EP-A1- 2 966 544
- WO-A1-2016/164917
- US-A1- 2017 188 066

## Beschreibung

Die vorliegende Erfindung betrifft ein Verfahren zur Darstellung einer virtuellen Realität bei dem mittels zumindest einer visuellen Darstellungseinheit eine visuelle virtuelle Realität an einem Darstellungsort dargestellt wird und bei dem mittels zumindest einer Erzeugungsvorrichtung zumindest die Umgebungseigenschaft Geruch und die Umgebungseigenschaft Windrichtung für die virtuelle Realität am Darstellungsort erzeugt wird.

Aus der DE 10 2018 103 572 A1 ist ein System zur Darstellung einer virtuellen Realität bekannt.

Aus der US 2017/0188066 A1 ist ein System zum Erleben von immersiven Sinneserfahrungen offenbart.

In der EP 2 966 544 A1 ist eine Vorrichtung zum Abgeben von Gerüchen für audiovisuelle Produkte beschrieben.

In der WO 2016/164917 A1 ist eine VR-Brille beschrieben, die ein Auslassteil umfasst, um Gerüche direkt zur Nase zu führen.

Aufgabe der vorliegenden Erfindung ist es somit, eine Darstellung der virtuellen Realität zu verbessern.

Die Aufgabe wird gelöst durch die Gegenstände mit den Merkmalen der unabhängigen Patentansprüche. Vorteilhafte Ausführungen sind in den abhängigen Ansprüchen definiert.

Vorgeschlagen wird ein Verfahren zur Darstellung einer virtuellen Realität. Mit Hilfe der virtuellen Realität können beispielsweise Orte dargestellt werden, welche nicht ohne größeren Aufwand erreicht werden. Beispielsweise können Naturorte dargestellt werden, welche am Darstellungsort nicht zu finden sind. Alternativ kann mittels der virtuellen Realität aber auch beispielsweise ein Museumsbesuch dargestellt werden, ohne selbst das Museum besuchen zu müssen. Mit Hilfe der virtuellen Realität können somit weit entfernte Orte, unzugängliche Orte oder sogar fiktive Orte zugänglich gemacht werden.

Beim Verfahren wird mittels zumindest einer visuellen Darstellungseinheit eine visuelle virtuelle Realität an einem Darstellungsort dargestellt. Mittels der visuellen virtuellen Realität werden beispielsweise Bilder dargestellt, die beispielsweise dem oben genannten Museum oder einer Naturumgebung entsprechen. Mittels der visuellen virtuellen Realität können zusätzlich oder alternativ auch bewegte Bilder dargestellt werden. Die visuelle virtuelle Realität entspricht dabei einer visuellen Darstellung des Naturortes oder des Museums.

Außerdem wird beim Verfahren mittels zumindest einer Erzeugungsvorrichtung zumindest die Umgebungseigenschaft Geruch und die Umgebungseigenschaft Windrichtung für die virtuelle Realität am Darstellungsort erzeugt. Mit Hilfe der zumindest einen Umgebungseigenschaft kann die virtuelle Realität noch realistischer ausgebildet werden. Neben der visuellen virtuellen Realität können damit noch weitere Sinneseindrücke am Darstellungsort für die virtuelle Realität erzeugt werden.

Erfindungsgemäß werden die zumindest von einem Aufnahmeort stammende Umgebungseigenschaft Geruch und Umgebungseigenschaft Windrichtung am Darstellungsort live empfangen. Der Aufnahmeort ist dabei zum Darstellungsort räumlich getrennt. Der Aufnahmeort ist gemäß dem oben genannten Beispiel beispielsweise ein Museum oder der weit abgelegene Naturort. Der Darstellungsort kann ferner eine Wohnung sein, also somit vom Museum räumlich getrennt sein.

Außerdem werden zumindest die Umgebungseigenschaft Geruch und die Umgebungseigenschaft Windrichtung am Darstellungsort mittels der Erzeugungsvorrichtung live erzeugt. Es wird somit am Darstellungsort die vom Aufnahmeort stammende Umgebungseigenschaft Geruch und Umgebungseigenschaft Windrichtung live empfangen und live erzeugt, so dass mittels dieser zumindest zwei Umgebungseigenschaften noch realistischere virtuelle Realität ausgebildet werden kann. Die Umgebungseigenschaft Geruch und die Umgebungseigenschaft Windrichtung, welche live zumindest empfangen und erzeugt werden, können auch als live Umgebungseigenschaften bezeichnet werden. Ein Benutzer erhält damit am Darstellungsort, also beispielsweise in seiner Wohnung, live die zumindest Umgebungseigenschaft Geruch und Umgebungseigenschaft Windrichtung welche im Moment am Aufnahmeort herrscht. Die virtuelle Realität wird somit noch realistischer. Es wird nicht nur die visuelle virtuelle Realität am Darstellungsort dargestellt, also beispielsweise die visuelle Umgebung im Museum mit den Gemälden oder Statuen, sondern auch die live Umgebungseigenschaften.

Vorteilhaft ist es, wenn visuelle Daten vom Aufnahmeort zur Darstellung der visuellen virtuellen Realität am Darstellungsort live empfangen und am Darstellungsort mittels der visuellen Darstellungseinheit live dargestellt werden. Zusätzlich oder alternativ können auch Audiodaten vom Aufnahmeort zur Darstellung von Tönen und/oder Sprache am Darstellungsort live empfangen und am Darstellungsort mittels zumindest einem Lautsprecher live dargestellt werden. Zusätzlich oder alternativ können auch akustische Daten vom Aufnahmeort zur Darstellung einer akustischen virtuellen Realität am Darstellungsort live empfangen und am Darstellungsort mittels einer akustischen Darstellungseinheit, beispielsweise einem Lautsprecher, live dargestellt werden.

Von Vorteil ist es, wenn die zumindest Umgebungseigenschaft Geruch und Umgebungseigenschaft Windrichtung vom Aufnahmeort an den Darstellungsort live übermittelt werden kann. Die zumindest Umgebungseigenschaft Geruch und Umgebungseigenschaft Windrichtung werden somit live übermittelt, live am Darstellungsort empfangen und live am Darstellungsort erzeugt. Zusätzlich können auch die visuellen Daten zur Darstellung der visuellen virtuellen Realität live übermittelt, live am Darstellungsort empfangen und live am Darstellungsort dargestellt bzw. erzeugt werden.

Als Umgebungseigenschaften am Aufnahmeort werden Windrichtung und Geruch erfasst, und von Vorteil ist es wenn zusätzlich eine Temperatur und/oder eine Luftfeuchtigkeit erfasst wird. Es werden am Darstellungsort Wind und Geruch entsprechend der am Aufnahmeort erfassten Umgebungseigenschaft erzeugt. Zusätzlich oder alternativ ist es von Vorteil, wenn am Darstellungsort Temperatur und/oder Luftfeuchtigkeit entsprechend der am Aufnahmeort erfassten Umgebungseigenschaft erzeugt werden. Weiterhin zusätzlich oder alternativ können auch eine Helligkeit und/oder Lichtverhältnisse erfasst und/oder am Darstellungsort erzeugt werden. Dadurch kann die virtuelle Realität realistischer ausgebildet werden. Der Benutzer sieht damit nicht nur mehr die Umgebung bzw. den Aufnahmeort bzw. eine Darstellung des Aufnahmeorts, sondern der Benutzer kann den Aufnahmeort, mit Windrichtung und Geruch, fühlen.

Vorteilhaft ist es, wenn die zumindest Umgebungseigenschaft Geruch und Umgebungseigenschaft Windrichtung mittels zumindest zwei Umgebungssensoren am Aufnahmeort erfasst wird. Die zwei Umgebungssensoren sind ein Windrichtungssensor und ein Geruchssensor. Ein weiterer Umgebungssensor kann beispielsweise ein Temperatursensor, ein Luftfeuchtigkeitssensor, ein Helligkeitssensor und/oder ein Lichtsensor sein.

Die virtuelle Realität wird mittels einer Steuereinheit dargestellt, wird,wobei die Steuereinheit die zumindest Umgebungseigenschaft Geruch und Umgebungseigenschaft Windrichtung vorzugsweise mittels einer Datenschnittstelle empfängt. Mittels der Datenschnittstelle wird insbesondere die zumindest Umgebungseigenschaft Geruch und Umgebungseigenschaft Windrichtung live empfangen. Die Datenschnittstelle kann somit Daten zumindest empfangen.

Vorteilhaft ist es, wenn die Erfassung zumindest der Umgebungseigenschaft Geruch und der Umgebungseigenschaft Windrichtung am Aufnahmeort mittels Anweisungen vom Darstellungsort angewiesen wird. Beispielsweise kann die Datenschnittstelle auch dazu ausgebildet sein, Daten, also beispielsweise die Anweisungen, zu versenden. Mittels der Datenschnittstelle können Daten bidirektional übermittelt werden. Beispielsweise kann ein Benutzer am Darstellungsort Anweisungen an den Aufnahmeort übermitteln, dass eine Blickrichtung geändert wird, so dass der Benutzer am Darstellungsort einen anderen Bereich im Museum sieht.

Von Vorteil ist es, wenn die Steuereinheit die zumindest Umgebungseigenschaft Geruch und Umgebungseigenschaft Windrichtung vom Aufnahmeort anfordert. Zusätzlich oder alternativ kann, insbesondere mittels der Steuereinheit, die Erfassung der zumindest Umgebungseigenschaft Geruch und Umgebungseigenschaft Windrichtung am Aufnahmeort gesteuert und/oder angewiesen werden. Beispielsweise kann der Benutzer vom Darstellungsort aus am Aufnahmeort anweisen, dass eine Blickrichtung geändert wird oder dass sich am Naturort in eine von ihm gewählte Richtung bewegt wird. Bei der Bewegung am Naturort können sich natürlich auch die Umgebungseigenschaft Geruch und die Umgebungseigenschaft Windrichtung ändern, wobei die zur Bewegung passende bzw. ändernde Umgebungseigenschaft am Darstellungsort dargestellt werden kann.

Vorteilhaft ist es, wenn die Steuereinheit die zumindest Umgebungseigenschaft Geruch und Umgebungseigenschaft Windrichtung mittels der zumindest zwei Umgebungssensoren am Aufnahmeort ermittelt. Zusätzlich kann die Steuereinheit auch mittels einer Kamera als Umgebungssensor, mittels der die visuellen Daten zur Darstellung der visuellen virtuellen Realität erfasst werden, eine Helligkeit am Aufnahmeort ermitteln.

Die Erzeugungsvorrichtung umfasst mehrere Ventilatoren, mittels denen die Windrichtung erzeugt wird. Außerdem umfasst die Erzeugungsvorrichtung mehrere Düsen, mittels denen ein Geruchsstoff abgegeben wird, der den Geruch erzeugt. Der Geruchsstoff kann ferner im Bereich der Ventilatoren abgegeben werden, so dass der Geruchsstoff zum Benutzer gelangt. Außerdem wird damit auch ein Ort der Geruchsentstehung bzw. eine Richtung, aus der der Geruch kommt, abgebildet. Weiterhin sind mehrere Ventilatoren und mehrere Düsen vorhanden, so dass eine Richtung des Windes und des Geruchs dargestellt wird. Die Ventilatoren und die Düsen dabei in der Stärke regelbar und/oder schwenkbar sein. Insbesondere mittels der Steuerung der Stärke der Ventilatoren kann Windstärke als Umgebungseigenschaft dargestellt werden. Die Erzeugungsvorrichtung, die den Geruch erzeugt, kann auch als Geruchsvorrichtung bezeichnet werden bzw. kann eine Erzeugungsvorrichtung sein.

Vorteilhaft ist es, wenn die Erzeugungsvorrichtung eine Geruchsvorrichtung umfasst, in dem vorzugsweise aus mehreren Basisstoffen der Geruchsstoff bzw. der Geruch gemischt wird. Dadurch können eine Vielzahl an Gerüchen erzeugt werden. Wie bereits beschrieben, kann die Erzeugungsvorrichtung auch die Geruchsvorrichtung sein. Zusätzlich oder alternativ kann die Erzeugungsvorrichtung bzw. die Geruchsvorrichtung auch die Geruchsstoffe umfassen, aus denen unmittelbar der Geruch ausgebildet wird.

Vorgeschlagen wird ferner ein System zur Darstellung einer virtuellen Realität laut Anspruch 7.

Das System umfasst zumindest eine visuelle Darstellungseinheit zur Darstellung einer visuellen virtuellen Realität an einem Darstellungsort. Die virtuelle Realität kann beispielsweise eine Darstellung eines Museums oder eines Naturortes sein, welche in einer privaten Wohnung als Darstellungsort dargestellt werden. Die Vorteile sind dabei, dass mittels der virtuellen Realität auch räumlich weit entfernte Orte, unzugängliche Orte oder sogar fiktive Orte besucht werden können, ohne dabei die eigene Wohnung verlassen zu müssen.

Das System umfasst außerdem zumindest eine Erzeugungsvorrichtung zur Erzeugung von zumindest der Umgebungseigenschaft Geruch und der Umgebungseigenschaft Windrichtung für die virtuelle Realität am Darstellungsort. Mit Hilfe der Umgebungseigenschaft Geruch und der Umgebungseigenschaft Windrichtung kann die virtuelle Realität noch realistischer dargestellt werden.

Weiterhin sind die zumindest eine von einem Aufnahmeort stammende Umgebungseigenschaft Geruch und Umgebungseigenschaft Windrichtung am Darstellungsort live empfangbar. Ferner ist die zumindest Umgebungseigenschaft Geruch und Umgebungseigenschaft Windrichtung mittels der zumindest einen Erzeugungsvorrichtung live erzeugbar. Die Umgebungseigenschaft Geruch und Umgebungseigenschaft Windrichtung sind somit am Darstellungsort live empfangbar und erzeugbar. Infolgedessen kann die virtuelle Realität realistischer ausgebildet werden.

Das System umfasst eine Steuereinheit, mittels der die virtuelle Realität darstellbar ist. Mittels der Steuereinheit sind dabei die visuelle virtuelle Realität darstellbar und die Umgebungseigenschaften mittels der Erzeugungsvorrichtung erzeugbar.

Vorteilhaft ist es, wenn die Steuereinheit mittels einer Datenschnittstelle die die zumindest Umgebungseigenschaft Geruch und Umgebungseigenschaft Windrichtung live empfangen und die zumindest Umgebungseigenschaft Geruch und Umgebungseigenschaft Windrichtung mittels der zumindest einen Erzeugungsvorrichtung live erzeugen kann.

Von Vorteil ist es, wenn die Steuereinheit die Erfassung der zumindest Umgebungseigenschaft Geruch und Umgebungseigenschaft Windrichtung am Aufnahmeort, insbesondere mittels der Datenschnittstelle, steuern kann. Zusätzlich oder alternativ ist es von Vorteil, wenn die Steuereinheit die zumindest Umgebungseigenschaft Geruch und Umgebungseigenschaft Windrichtung vom Aufnahmeort, insbesondere mittels der Datenschnittstelle, anfordern kann. Dadurch kann die virtuelle Realität vom Darstellungsort gesteuert werden. Die Steuereinheit ist dabei vorzugsweise am Darstellungsort angeordnet.

Vorteilhaft ist es, wenn mittels der Steuereinheit die zumindest Umgebungseigenschaft Geruch und Umgebungseigenschaft Windrichtung von zumindest zwei Umgebungssensoren am Aufnahmeort ermittelbar sind.

Die Steuereinheit kann dabei derart ausgebildet sein, dass diese die zumindest Umgebungseigenschaft Geruch und Umgebungseigenschaft Windrichtung mit Hilfe der Umgebungssensoren am Aufnahmeort ermitteln kann. Zusätzlich kann die Steuereinheit mittels einer Kamera als Umgebungssensor eine Helligkeit bzw. Lichtverhältnisse ermitteln. Die Steuereinheit kann mittels eines Windsensors als Umgebungssensor neben der Windstärke auch eine Windrichtung ermitteln.

Von Vorteil ist es, wenn die Steuereinheit mittels der Datenschnittstelle eine Verbindung zu den zumindest zwei Umgebungssensoren am Aufnahmeort ausbilden kann, um die zumindest Umgebungseigenschaft Geruch und Umgebungseigenschaft Windrichtung live empfangen und/oder die zumindest Umgebungseigenschaft Geruch und Umgebungseigenschaft Windrichtung ermitteln zu können.

Die Erzeugungsvorrichtung umfasst mehrere Ventilaor und mehrere Düsen, Mit Hilfe der Ventilatoren wird Windrichtung als Umgebungseigenschaft erzeugt. Die Ventilatoren können ferner in der Stärke regelbar sein, so dass auch eine Windstärke als Umgebungseigenschaft erzeugbar ist. Die Erzeugungsvorrichtung umfasst die Geruchsvorrichtung. Geruchsvorrichtung kann ferner die Behälter für die Basisstoffe und/oder Behälter mit den Geruchsstoffen umfassen. Vorteilhaft ist es ferner, wenn die Behälter austauschbar sind, so dass leere Behälter gegen neue Behälter ausgetauscht werden können.

Von Vorteil ist es, wenn die Geruchsvorrichtung zur Bevorratung von Basisstoffen mehrere Behälter umfasst, wobei die Geruchsvorrichtung die Gerüche mittels der Basisstoffe mischen kann. Dadurch kann mit einer geringen Anzahl an verschiedenen Basisstoffen eine Vielzahl an Gerüchen gemischt werden. Zusätzlich oder alternativ kann die Erzeugungsvorrichtung bzw. die Geruchsvorrichtung auch die Geruchsstoffe umfassen, aus denen unmittelbar der Geruch ausgebildet wird. Die Erzeugungsvorrichtung bzw. die Geruchsvorrichtung kann ferner die Behälter für die Basisstoffe und/oder Behälter mit den Geruchsstoffen umfassen. Vorteilhaft ist es ferner, wenn die Behälter austauschbar sind, so dass leere Behälter gegen neue Behälter ausgetauscht werden können.

Das System kann ferner eine visuelle Erfassungseinheit umfassen, mittels der die visuellen Daten zur Darstellung der visuellen virtuellen Realität am Aufnahmeort erfasst werden können. Zusätzlich kann das System auch zumindest ein Mikrofon umfassen, um Schall am Aufnahmeort erfassen zu können.

Die Geruchsvorrichtung umfasst zumindest einen Behälter zur Bevorratung zumindest eines Geruchsstoffs. Zusätzlich oder alternativ kann die Geruchsvorrichtung auch zumindest einen Behälter umfassen, in dem ein Basisstoff enthalten ist, mittels dem der Geruchsstoff ausgebildet, insbesondere gemischt, werden kann.

Von Vorteil ist es, wenn die Geruchsvorrichtung zur Bevorratung von Basisstoffen und/oder Geruchsstoffen mehrere Behälter umfasst. Ferner kann die Geruchsvorrichtung die Gerüche mittels den Basisstoffen mischen. Dazu kann die Geruchsvorrichtung eine Mischvorrichtung aufweisen.

Von Vorteil ist es, wenn die Behälter austauschbar sind. Dadurch können volle Behälter eingesetzt werden, wenn die Behälter leer sind.

Mit Hilfe der Düsen kann der Geruchsstoff und/oder die Basisstoffe, die beispielsweise an der Luft die Geruchsstoffe ausbilden können, abgegeben werden. Zumindest zwei der Ventilatoren sind jeweils einer Düse zugeordnet, so dass die Ventilatoren die Geruchsstoffe am Darstellungsort verteilt bzw. zum Benutzer befördert.

Die Steuereinheit ist derart ausgebildet, dass mittels dieser eine Geruchsumgebung eingestellt werden kann. In der Steuereinheit oder in einem mit der Steuereinheit verbundenen Speicher, können verschiedene Geruchsumgebungen bzw. Geruchsszenarien hinterlegt sein, die ausgewählt werden können. Beispielsweise kann als Geruchsumgebungen bzw. Geruchsszenarien eine Blumenwiese gespeichert sein, so dass bei der virtuellen Realität das Geruchsempfinden einer Blumenwiese erlebt werden kann.

Die Steuereinheit kann auch eine Datenschnittstelle aufweisen, mittels der die Umgebungseigenschaften Geruch und Windrichtung vom Aufnahmeort, insbesondere live, empfangen werden können. Dadurch können die Gerüche am Darstellungsort an die Gerüche am Aufnahmeort angepasst werden. Dabei kann es auch ausreichen, wenn lediglich Positionsdaten vom Aufnahmeort an die Steuereinheit übermittelt werden. Die Steuereinheit kann dann, insbesondere selbstständig, ermitteln, welche Gerüche am Aufnahmeort vorhanden sind und die entsprechenden Gerüche am Darstellungsort erzeugen. Beispielsweise ermittelt die Steuereinheit, dass der Aufnahmeort auf einer Blumenwiese ist und kann dann entsprechende Gerüche am Darstellungsort erzeugen.

Weitere Vorteile der Erfindung sind in den nachfolgenden Ausführungsbeispielen beschrieben. Es zeigen:
- Figur 1: eine schematische Ansicht eines Systems zur Darstellung einer virtuellen Realität und
- Figur 2: eine schematische Ansicht einer Geruchsvorrichtung.

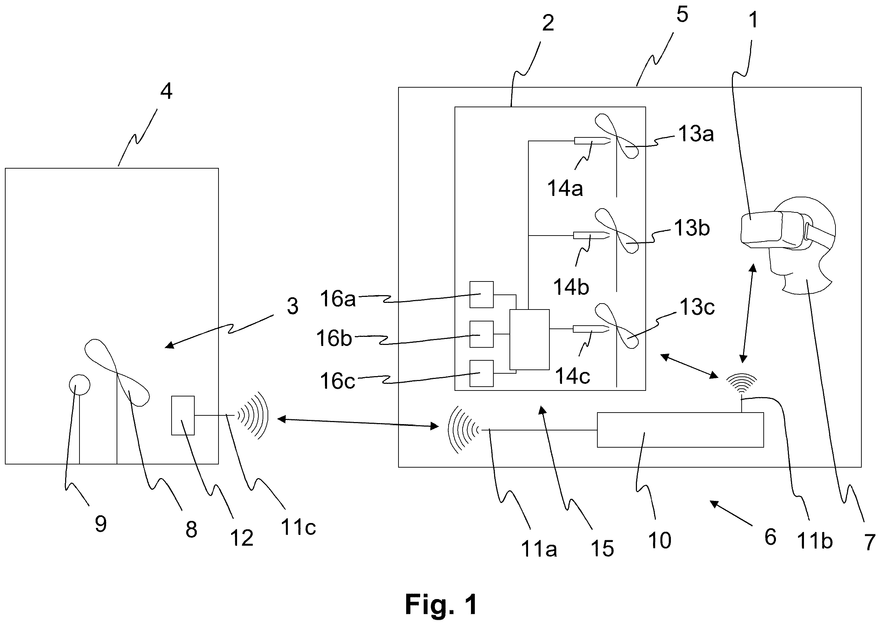

Die Erfindung wird am besten im Lichte der Ausführungsform verstanden, die im Zusammenhang mit Fig. 1 beschrieben ist.

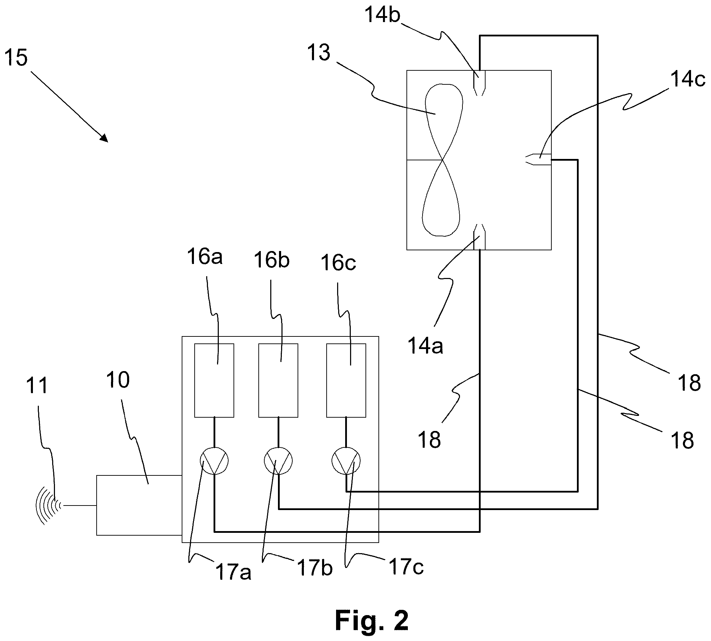

Die in Figur 2 beschriebene Ausführungsform beschreibt nicht die vollständige Kombination von Merkmalen, wie beansprucht, sondern ist zum Verständnis der beanspruchten Erfindung nützlich. U

Figur 1 zeigt eine schematische Ansicht eines Systems 6 zur Darstellung einer virtuellen Realität.

Das System 6 umfasst zumindest eine visuelle Darstellungseinheit 1, mittels der eine visuelle virtuelle Realität dargestellt wird. Die visuelle Darstellungseinheit 1 ist in diesem Ausführungsbeispiel als eine Brille, eine sogenannte VR-Brille (virtuelle Realität Brille) ausgebildet, welche von einem Benutzer 7 getragen wird. Alternativ kann die visuelle Darstellungseinheit 1 auch ein Monitor, ein Bildschirm oder ein Projektor sein. Mit Hilfe der visuellen Darstellungseinheit 1 kann eine visuelle virtuelle Realität dargestellt werden. Diese visuelle virtuelle Realität kann Bilder oder Videos umfassen. Mittels der visuellen Darstellungseinheit 1 kann die visuelle virtuelle Realität an einem Darstellungsort 5 dargestellt werden. Der Darstellungsort 5 ist dabei der Ort, an dem sich der Benutzer 7 befindet. Beispielsweise ist der Darstellungsort 5 eine Wohnung, an dem die virtuelle Realität dargestellt wird. Der Vorteil der virtuellen Realität liegt darin, dass damit beispielsweise ein Museumsbesuch durchgeführt werden kann, ohne das Museum besuchen zu müssen. Der hier beschriebene Museumsbesuch kann damit bequem von zu Hause erfolgen. Als weiteres Beispiel können damit auch Naturorte besucht werden, welche weit entfernt, unzugänglich oder rein fiktiv sind. Beispielsweise kann mit Hilfe der virtuellen Realität auch ein Meer oder ein Ozean besucht werden. Zusätzlich können für die virtuelle Realität auch passende Töne und/oder Sprache erzeugt werden.

Des Weiteren umfasst das System 6 zumindest eine Erzeugungsvorrichtung 2 zum Erzeugen von zumindest der Umgebungseigenschaft Geruch und der Umgebungseigenschaft Windrichtung, für die virtuelle Realität am Darstellungsort 5. Zusätzliche Umgebungseigenschaften sind beispielsweise Windstärke, Temperatur, Luftfeuchtigkeit, Helligkeit und/oder Lichtverhältnisse. Mit Hilfe der Erzeugung der Umgebungseigenschaft Geruch und Umgebungseigenschaft Windrichtung, kann die virtuelle Realität noch realistischer dargestellt werden. Beispielsweise kann damit der Besuch an einem Ozean mittels der visuellen Darstellungseinheit 1 nicht nur visuell dargestellt werden, sondern es kann auch zumindest die Umgebungseigenschaft Geruch und die Umgebungseigenschaft Windrichtung, erzeugt werden. Am Ozean kann somit nicht nur der Blick auf den Ozean dargestellt werden, sondern auch ein auf- oder ablandiger Wind und ein entsprechender salziger Geruch.

Beim Verfahren zur Darstellung der virtuellen Realität werden die zumindest zwei von einem Aufnahmeort 4 stammenden Umgebungseigenschaft Geruch und Umgebungseigenschaft Windrichtung am Darstellungsort 5 live empfangen. Ferner wird beim Verfahren die zumindest Umgebungseigenschaft Geruch und Umgebungseigenschaft Windrichtung am Darstellungsort 5 mittels der Erzeugungsvorrichtung 2 live erzeugt. Die vom Aufnahmeort 4 stammenden Umgebungseigenschaft Geruch und Umgebungseigenschaft Windrichtung werden somit am Darstellungsort 5 live empfangen und erzeugt. Somit wird mittels der virtuellen Realität die zumindest Umgebungseigenschaft Geruch und die Umgebungseigenschaft Windrichtung am Darstellungsort 5 dargestellt, welche im Moment am Aufnahmeort 4 herrscht. Es wird somit eine live Umgebungseigenschaft Geruch und eine live Umgebungseigenschaft Windrichtung am Darstellungsort 5 dargestellt. Die Umgebungseigenschaft Geruch und die Umgebungseigenschaft Windrichtung werden somit am Darstellungsort 5 in Echtzeit empfangen und erzeugt. Die live Umgebungseigenschaften passen ferner zu den gerade dargestellten visuellen Daten und/oder zu den dargestellten Tönen. Werden beispielsweise sich aufgrund von Wind bewegende Bäume visuell dargestellt, wird ebenfalls der entsprechende Wind als Umgebungseigenschaft erzeugt. Die visuellen Daten, die Töne bzw. der Schall und die zumindest erzeugte Umgebungseigenschaft Geruch und erzeugte Umgebungseigenschaft Windrichtung passen jeweils zueinander. Die entsprechende visuelle virtuelle Realität, die akustische virtuelle Realität und die zu der Umgebungseigenschaft entsprechende virtuelle Realität passen somit ebenfalls zueinander.

Die zumindest eine Umgebungseigenschaft Geruch und eine Umgebungseigenschaft Windrichtung können dabei auch vom Aufnahmeort 4 an den Darstellungsort 5 live übermittelt werden Gemäß dem vorliegenden Ausführungsbeispiel ist am Aufnahmeort 4 schematisch dargestellt zumindest zwei Umgebungssensoren 8 und 9 angeordnet. Mit Hilfe des zumindest einen Geruchssensors 9 und Windsensors 8 können die zumindest Umgebungseigenschaft Geruch und Umgebungseigenschaft Windrichtung am Aufnahmeort 4 erfasst werden. Mittels des Umgebungssensors 3 können die Umgebungseigenschaft Geruch und die Umgebungseigenschaft Windrichtung am Aufnahmeort 4 ermittelt werden. Hier sind als Umgebungssensoren 3 ein Windsensor 8 und ein Geruchssensor 9 dargestellt. Es können auch mehrere entsprechende Sensoren 3 verwendet werden bzw. angeordnet sein. Zusätzlich können auch ein Temperatur-, ein Licht-, ein Helligkeits- und/oder ein Feuchtigkeitssensor vorhanden sein.

Des Weiteren umfasst das System 6 gemäß dem vorliegenden Ausführungsbeispiel eine Steuereinheit 10, mittels dem die Darstellung der virtuellen Realität gesteuert wird bzw. werden kann.

Die Steuereinheit 10 umfasst weiterhin gemäß dem vorliegenden Ausführungsbeispiel zumindest eine Datenschnittstelle 11a, 11b. Mit Hilfe der zumindest einen Datenschnittstelle 11a, 11b können zumindest die Umgebungseigenschaft Geruch und die Umgebungseigenschaft Windrichtung live empfangen werden. Die Umgebungseigenschaft Geruch und die Umgebungseigenschaft Windrichtung können dann live erzeugt werden. Um die Umgebungseigenschaft Geruch und die Umgebungseigenschaft Windrichtung live, insbesondere vom Aufnahmeort 4, empfangen zu können, weist die Steuereinheit 10 hier eine erste Datenschnittstelle 11a auf.

Mittels der ersten Datenschnittstelle 11a können die Umgebungseigenschaften live empfangen und/oder es kann eine Verbindung zum Aufnahmeort 4 ausgebildet werden, um die Umgebungseigenschaften empfangen zu können. Zusätzlich können mittels der ersten Datenschnittstelle 11a auch die visuellen Daten und optional die akustischen Daten zur Darstellung der visuellen virtuellen und optional akustischen Realität, vorzugsweise live**,** empfangen werden. Auch diese visuellen und optional akustischen Daten werden live mittels der visuellen Darstellungseinheit 1 dargestellt.

Um die Umgebungseigenschaft Geruch und die Umgebungseigenschaft Windrichtung an den Darstellungsort 5 übermitteln zu können, kann am Aufnahmeort 4 eine Kommunikationseinheit 12 angeordnet sein, welche, wie hier dargestellt ist, eine dritte Datenschnittstelle 11c aufweist. Die Kommunikationseinheit 12 kann ferner dazu ausgebildet sein, die vom Umgebungssensor 3 erfasste Umgebungseigenschaft Geruch und erfasste Umgebungseigenschaft Windrichtung zu sammeln und/oder auszuwerten. Die Kommunikationseinheit 12 kann außerdem die Umgebungseigenschaft Geruch und Umgebungseigenschaft Windrichtung mittels der dritten Datenschnittstelle 11c an den Darstellungsort 5, insbesondere live übermitteln. Dort kann die erste Datenschnittstelle 11a die Umgebungseigenschaft Geruch und Umgebungseigenschaft Windrichtung live empfangen. Eine hier lediglich mittels einem Pfeil dargestellte Verbindung zwischen dem Aufnahmeort 4 und dem Darstellungsort 5 bzw. zwischen der dritten und der ersten Datenschnittstelle 11c, 11a kann beispielsweise das Internet sein. Über dieselbe Verbindung können auch die visuellen Daten, insbesondere live, übermittelt werden.

Die hier gezeigte Steuereinheit 10 umfasst gemäß dem vorliegenden Ausführungsbeispiel eine zweite Datenschnittstelle 11b, mittels der eine mittels Pfeilen schematische Verbindung zur visuellen Darstellungseinheit 1 und zur Erzeugungsvorrichtung 2 ausgebildet werden kann. Alternativ kann die Steuereinheit 10 auch für die visuelle Darstellungseinheit 1 und für die Erzeugungsvorrichtung 2 jeweils eine eigene Datenschnittstelle 11 aufweisen.

Die zumindest zwei Umgebungseigenschaften Geruch und Windrichtung werden somit an die zumindest eine Erzeugungsvorrichtung 2 zur live-Erzeugung der Umgebungseigenschaft Geruch und Umgebungseigenschaft Windrichtung übermittelt.

Die Steuereinheit 10 kann ferner, insbesondere mittels der ersten Datenschnittstelle 11a, die Erfassung der visuellen und/oder der akustischen Daten und/oder der zumindest Umgebungseigenschaft Geruch und Umgebungseigenschaft Windrichtung steuern und/oder anfordern. Beispielsweise kann auch eine Person am Aufnahmeort 4 angewiesen werden, sich in eine Richtung zu drehen oder sich in eine Richtung zu bewegen. Beispielsweise kann am Aufnahmeort 4 auch eine Drohne angeordnet sein, welche die visuellen und/oder die akustischen Daten und/oder die zumindest Umgebungseigenschaft Geruch und Umgebungseigenschaft Windrichtung erfassen kann. Mittels der Steuereinheit 10 kann die Drohne bewegt werden. Zusätzlich oder alternativ zur Drohne kann am Aufnahmeort 4 auch ein steuerbares Fahrzeug oder ähnliches angeordnet sein.

Gemäß dem vorliegenden Ausführungsbeispiel umfasst die Erzeugungsvorrichtung 2 mehrere Ventilatoren 13a - 13c. Mit Hilfe der Ventilatoren 13a - 13c kann somit ein Wind als Umgebungseigenschaft erzeugt werden. Die Ventilatoren 13a - 13c können regelbar sein, um eine Windstärke darstellen zu können. Die Ventilatoren 13a - 13c können auch schwenkbar sein, um eine Windrichtung darstellen zu können. Gemäß dem vorliegenden Ausführungsbeispiel sind hier drei Ventilatoren 13a - 13c gezeigt, so dass infolgedessen der Wind aus mehreren Richtungen darstellbar ist. Die drei Ventilatoren 13a - 13c sind dabei in einer speziellen Anordnung zum Benutzer 7 angeordnet.

Des Weiteren umfasst die Erzeugungsvorrichtung 2 gemäß dem vorliegenden Ausführungsbeispiel mehrere Düsen 14a - 14c, mittels denen Geruchsstoffe am Darstellungsort 5 verteilt werden können, um einen Geruch als Umgebungseigenschaft darstellen zu können. Die Düsen 14a - 14c sind im Bereich der Ventilatoren 13a - 13c angeordnet, um den Geruchsstoff zum Benutzer 7 zu befördern. Gemäß dem vorliegenden Ausführungsbeispiel sind hier drei Dus̈en 14a - 14c gezeigt. Dadurch wird eine Richtungsabhängigkeit des Geruchs erreicht. Dem Benutzer 7 wird damit dargestellt, dass ein Geruch aus einer bestimmten Richtung kommt. Ferner ist hier den Ventilatoren 13a - 13c jeweils eine Düse 14a - 14c zugeordnet. Natürlich kann auch nur zwei der Ventilatoren 13a - 13c eine Düse 14a - 14c zugeordnet sein.

Die Erzeugungsvorrichtung 2 umfasst ferner eine Geruchsvorrichtung 15 zum Ausbilden der Gerüche. Die Geruchsvorrichtung 15 ist beispielsweise mittels Leitungen mit den mehreren Düsen 14a - 14c verbunden, um den Geruchsstoff zur Düse 14a - 14c leiten zu können.

Die Geruchsvorrichtung 15 weist weiterhin mehrere Behälter 16a - 16 c auf und/oder ist mit diesen gekoppelt, wobei in den Behältern 16a - 16c jeweils Basisstoffe für die Gerüche angeordnet sind. Die Geruchsvorrichtung 15 kann mittels den mehreren Basisstoffen die Geruchsstoffe mischen, so dass mehrere verschiedene Gerüche ausgebildet werden können.

Figur 2 zeigt eine schematische Ansicht der Geruchsvorrichtung 15 zum Erzeugen von Gerüchen am Darstellungsort 5. Die hier gezeigte Geruchsvorrichtung 15 kann auch die Erzeugungsvorrichtung 2 oder eine Ausführungsform der Erzeugungsvorrichtung 2 sein. Alternativ kann die Erzeugungsvorrichtung 2 die Geruchsvorrichtung 15 umfassen. Alternativ kann auch die Geruchsvorrichtung 15 die Erzeugungsvorrichtung 2 umfassen.

Merkmale, welche bereits in der vorgegangenen Figur beschrieben sind, können der Einfachheit halber nicht nochmals erklärt werden. Des Weiteren werden der Einfachheit halber für gleiche Merkmal gleiche Bezugszeichen verwendet.

Die Geruchsvorrichtung 15 umfasst zumindest einen Behälter 16a - 16c zur Bevorratung von Geruchsstoffen und/oder der Basisstoffe. Die Behälter 16a - 16c können austauschbar sein, um leere gegen neue Behälter 16a - 16c austauschen zu können oder um die Geruchsvorrichtung 15 mit neuen Gerüchen zu versorgen.

Des Weiteren umfasst die Geruchsvorrichtung 15 zumindest eine Verteilereinheit 13, 14, welche hier ein Ventilator 13 und/oder eine Düse 14 ist. Die Geruchsvorrichtung 15 kann dabei auch mehrere Ventilatoren 13 und/oder Düsen 14 aufweisen. Ferner kann jedem Ventilator 13 zumindest eine Düse 14 zugeordnet sein, um beim Erzeugen des Windes für die virtuelle Realität den Geruchsstoff abzugeben und um diesen mittels des Windes zu befördern.

Gemäß dem vorliegenden Ausführungsbeispiel sind dem Ventilator 13 drei Düsen 14a - 14c zugeordnet, um verschiedenen Geruchsstoffe abgeben zu können. Des Weiteren ist gemäß dem vorliegenden Ausführungsbeispiel jede Düse 14a - 14c mit jeweils einem Behälter 16a - 16c mittels Leitungen 18 verbunden. Die Geruchsvorrichtung 15 weist zumindest eine Pumpe 17a - 17c auf, um die Geruchsstoffe zu den Düsen 14a - 14c zu befördern. Hier ist jedem Behälter 16a - 16c und/oder jeder Düse 14a - 14c eine Pumpe 16a - 16c zugeordnet. Infolgedessen kann ein Vermischen der Geruchsstoffe aus den Behältern 16a - 16c vermieden werden, wenn beispielsweise in den Behältern 16a - 16c ein Geruchsstoff ist. Wenn dagegen in den Behältern 16a - 16c Basisstoffe für die Geruchsstoffe vorhanden sind, kann es vorteilhaft sein, wenn die Geruchsvorrichtung 15 eine Mischvorrichtung umfasst, um die Basisstoffe entsprechend zu mischen. In diesem Fall können die Pumpen 17a - 17c die Basisstoffe aus den Behältern 16a - 16c in die Mischvorrichtung pumpen. Alternativ können die Basisstoffe auch zum Ventilator 13 geleitet werden, wobei sich diese in der dortigen Luftströmung zum Geruchsstoff vermischen.

Gemäß dem vorliegenden Ausführungsbeispiel ist der Geruchsvorrichtung 15 die Steuereinheit 10 zugeordnet. Diese kann die Steuereinheit 10 der Figur 1 oder eine zusätzliche Steuereinheit 10 sein, die der Geruchsvorrichtung 15 zugeordnet ist oder die die Geruchsvorrichtung 15 umfasst.

Die Geruchsvorrichtung 15 bzw. die Steuereinheit 10 weist eine Datenschnittstelle 11 auf, um Daten vom Aufnahmeort 4, insbesondere live, empfangen zu können. Mittels dieser Daten vom Aufnahmeort 4 kann daraufhin ein zum Aufnahmeort 4 passender Geruch für die virtuelle Realität mittels der Geruchsvorrichtung 15 erzeugt werden.

Die hier gezeigte Steuereinheit 10, wie auch die Steuereinheit 10 der Figur 1, kann auch einen, hier nicht gezeigte, Speicher umfassen, um Geruchsinformationen zu speichern. Mit Hilfe des Speichers bzw. der Geruchsinformationen können die zum Aufnahmeort 4 passenden Gerüche erzeugt werden. Beispielsweise können Daten über die Gerüche an die Steuereinheit 10 übermittelt werden, welche dann erzeugt werden. Zusätzlich oder alternativ können auch Daten über den Aufnahmeort 4 übermittelt werden, anhand derer dann entsprechende Gerüche erzeugt werden. Beispielsweise werden Daten übermittelt, dass am Aufnahmeort 4 eine Blumenwiese vorhanden ist. Anhand dieser Daten kann dann ein entsprechender Geruch erzeugt werden. Zusätzlich oder alternativ können auch GPS-Daten übermittelt werden. Anhand der GPS-Daten kann dann die Steuereinheit 10 ermitteln, welche Gerüche am Aufnahmeort 4 vorhanden sind, woraufhin dann die Gerüche erzeugt werden.

### Bezugszeichenliste

- 1: visuelle Darstellungseinheit
- 2: Erzeugungsvorrichtung
- 3: Umgebungssensor
- 4: Aufnahmeort
- 5: Darstellungsort
- 6: System
- 7: Benutzer
- 8: Windsensor
- 9: Geruchssensor
- 10: Steuereinheit
- 11: Datenschnittstelle
- 12: Kommunikationseinheit
- 13: Ventilator
- 14: Düse
- 15: Geruchsvorrichtung
- 16: Behälter
- 17: Pumpe
- 18: Leitung

## Patentansprüche

1. Verfahren zur Darstellung einer virtuellen Realität, für ein System (6) zur Darstellung der virtuellen Realität,
bei dem mittels zumindest einer visuellen Darstellungseinheit (1) des Systems (6) eine virtuelle Realität visuell an einem Darstellungsort (5) dargestellt wird,
bei dem zumindest eine von einem Aufnahmeort (4) stammende Umgebungseigenschaft am Darstellungsort (5) live empfangen wird und
bei dem die zumindest eine Umgebungseigenschaft für die virtuelle Realität am Darstellungsort (5) mittels zumindest einer Erzeugungsvorrichtung (2) des Systems (6) live erzeugt wird wobei die Erzeugungsvorrichtung (2) Mittel (13, 14), die Geruchsstoff abgeben, umfasst, um einen Geruch als erste Umgebungseigenschaft darzustellen, und
wobei die Darstellung der virtuellen Realität mittels einer Steuereinheit (10) des Systems (6) gesteuert wird, H
**dadurch gekennzeichnet,**
**dass** die Erzeugungsvorrichtung (2) als Mittel (13, 14) mehrere Ventilatoren (13a, 13b, 13c) fasst, mittels denen als zweite Umgebungseigenschaft am Darstellungsort (5) eine Windrichtung dargestellt wird, so dass ein Wind aus mehreren Richtungen dargestellt wird,
**dass** die Erzeugungsvorrichtung (2) als Mittel (13, 14) zusätzlich mehrere Düsen (14a, 14b, 14c) umfasst, um den Geruch als erste Umgebungseigenschaft darzustellen, wobei mittels jeder Düse (14a, 14b, 14c) ein Geruchsstoff abgegeben wird, und
**dass** zumindest zwei der Ventilatoren (13) jeweils eine Düse (14) zugeordnet ist, um eine Richtungsabhängigkeit des Geruchs zu erreichen, so dass dem Benutzer (7) dargestellt wird, dass ein Geruch aus einer bestimmten Richtung kommt.

2. Verfahren nach dem vorherigen Anspruch, **dadurch gekennzeichnet, dass** visuelle Daten vom Aufnahmeort (4) zur Darstellung der visuellen virtuellen Realität am Darstellungsort (5) live empfangen und am Darstellungsort (5) mittels der visuellen Darstellungseinheit (1) live dargestellt werden.

3. Verfahren nach einem der vorherigen Ansprüche **dadurch gekennzeichnet, dass** als Umgebungseigenschaft am Aufnahmeort (4) Windrichtung und Geruch erfasst wird, und eine Temperatur und/oder eine Luftfeuchtigkeit erfasst wird, und/oder dass die zumindest erste Umgebungseigenschaft und zweite Umgebungseigenschaft mittels zwei Umgebungssensoren (8, 9, 3) am Aufnahmeort (4) erfasst werden,
und/oder dass am Darstellungsort (5) Windrichtung und Geruch erfasst wird, und Temperatur und/oder Luftfeuchtigkeit entsprechend der am Aufnahmeort (4) erfassten Umgebungseigenschaften erzeugt wird.

4. Verfahren nach einem der vorherigen Ansprüche,
**dadurch gekennzeichnet, dass** die Steuereinheit (10) die zumindest erste Umgebungseigenschaft und zweite Umgebungseigenschaft vorzugsweise mittels einer Datenschnittstelle (11) empfängt, und/oder dass die Steuereinheit (10) die zumindest erste Umgebungseigenschaft und zweite Umgebungseigenschaft vom Aufnahmeort (4) anfordert und/oder
dass, insbesondere mittels der Steuereinheit (10), die Erfassung der zumindest ersten Umgebungseigenschaft und zweiten Umgebungseigenschaft am Aufnahmeort (4) und/oder die Erfassung der visuellen Daten gesteuert und/oder angewiesen wird.

5. Verfahren nach einem der vorherigen Ansprüche **dadurch gekennzeichnet, dass** die Erfassung der zumindest ersten Umgebungseigenschaft und zweiten Umgebungseigenschaft am Aufnahmeort (4) mittels Anweisungen vom Darstellungsort (5) angewiesen wird.

6. Verfahren nach einem der vorherigen Ansprüche, **dadurch gekennzeichnet, dass** mittels den Düsen (14a, 14b, 14c) der Geruchsstoff abgegeben wird, der den Geruch erzeugt und dass der Geruchsstoff im Bereich des Ventilators (13) abgegeben wird und/oder
dass die Erzeugungsvorrichtung (2) eine Geruchsvorrichtung (15) umfasst, in der vorzugsweise aus mehreren Basisstoffen der Geruchsstoff gemischt wird.

7. System (6) zur Darstellung einer virtuellen Realität, wobei das System (6) insbesondere derart ausgebildet ist, dass es gemäß einem Verfahren nach einem der vorherigen Ansprüche betreibbar ist,
mit zumindest einer visuellen Darstellungseinheit (1) zur visuellen Darstellung einer virtuellen Realität an einem Darstellungsort (5) und mit zumindest einer Erzeugungsvorrichtung (2) zur live Erzeugung von zumindest einer Umgebungseigenschaft für die virtuelle Realität am Darstellungsort (5),
wobei die zumindest eine von einem Aufnahmeort (4) stammende Umgebungseigenschaft am Darstellungsort (5) live empfangen wird und die zumindest eine Umgebungseigenschaft mittels der zumindest einen Erzeugungsvorrichtung (2) live erzeugt wird, wobei die Erzeugungsvorrichtung (2) Mittel (13, 14), die Geruchsstoff abgeben, umfasst, um einen Geruch als erste Umgebungseigenschaft darzustellen, und H
mit einer Steuereinheit (10) mittels der die Darstellung der virtuellen Realität gesteuert wird,
**dadurch gekennzeichnet**, H
dass die Erzeugungsvorrichtung (2) als Mittel (13, 14) mehrere Ventilatoren (13a, 13b, 13c) um-fasst, mittels denen als zweite Umgebungseigenschaft am Darstellungsort (5) eine Windrichtung dargestellt wird, so dass ein Wind aus mehreren Richtungen dargestellt werden kann,
dass die Erzeugungsvorrichtung (2) als Mittel (13, 14) zusätzlich mehrere Düsen (14a, 14b, 14c) umfasst, um den Geruch als erste Umgebungseigenschaft darzustellen, wobei mittels jeder Düse (14a, 14b, 14c) ein Geruchsstoff abgegeben wird, und
dass zumindest zwei der Ventilatoren (13) jeweils eine Düse (14) zugeordnet ist, um eine Richtungsabhängigkeit des Geruchs zu erreichen, so dass dem Benutzer (7) dargestellt werden kann, dass ein Geruch aus einer bestimmten Richtung kommt.

8. System nach dem vorherigen Anspruch 7, **dadurch gekennzeichnet,**
**dass** die Steuereinheit (10) mittels einer Datenschnittstelle (11) die zumindest erste Umgebungseigenschaft und zweite Umgebungseigenschaft vorzugsweise live empfangen kann und/oder
**dass** die Steuereinheit (10) mittels der Datenschnittstelle (11) eine Verbindung zu zumindest zwei Umgebungssensoren (8, 9, 3) am Aufnahmeort (4) vorzugsweise ausbilden kann, um die zumindest erste Umgebungseigenschaft und zweite Umgebungseigenschaft live zu empfangen und/oder die zumindest erste Umgebungseigenschaft und zweite Umgebungseigenschaft ermitteln zu können.

9. System nach einem der vorherigen Ansprüche 7 oder 8, **dadurch gekennzeichnet, dass** die Steuereinheit (10) die Erfassung der zumindest ersten Umgebungseigenschaft und zweiten Umgebungseigenschaft am Aufnahmeort (4), insbesondere mittels der Datenschnittstelle (11), steuern kann und/oder dass die zumindest erste Umgebungseigenschaft und zweite eft-re- Umgebungseigenschaft vom Aufnahmeort (4), insbesondere mittels der Datenschnittstelle (11), anfordern kann.

10. System nach einem der vorherigen Ansprüche 7 bis 9, **dadurch gekennzeichnet, dass** mittels der Steuereinheit (10) die zumindest erste Umgebungseigenschaft und zweite Umgebungseigenschaft von zumindest zwei Sensoren (8,9,3) am Aufnahmeort (4) ermittelbar ist.

11. System nach einem der vorherigen Ansprüche 7 bis 10,
**dadurch gekennzeichnet, dass** die Erzeugungsvorrichtung (2) eine Geruchsvorrichtung (15) zur Erzeugung von Gerüchen für eine virtuelle Realität umfasst, wobei die Geruchsvorrichtung (15) zur Bevorratung von Basisstoffen mehrere Behälter (16) umfasst, wobei die Geruchsvorrichtung (15) die Gerüche mittels den Basisstoffen mischen kann, und/oder
dass die Geruchsvorrichtung (15) mehrere, insbesondere austauschbare, Behälter (16) umfasst.

12. System nach dem vorherigen Anspruch 7, **dadurch gekennzeichnet, dass** die Erzeugungsvorrichtung (2) eine Geruchsvorrichtung (15) zur Erzeugung von Gerüchen für eine virtuelle Realität umfasst, wobei die Geruchsvorrichtung (15) zumindest einen Behälter (16) zur Bevorratung eines Geruchsstoffs und/oder Basisstoffen umfasst.

13. System nach dem vorherigen Anspruch, **dadurch gekennzeichnet, dass** die Geruchsvorrichtung (15) zur Bevorratung von Basisstoffen und/oder Geruchsstoffen mehrere Behälter (16) umfasst und/oder dass die Behälter (16) austauschbar sind.

## Claims

1. A method for representation a virtual reality, for a system (6) for representing the virtual reality,
in which a visual virtual reality is represented visually at a display location (5) by means of at least one visual display unit (1) of the system (6)
in which at least one surroundings property, which originates from a recording location (4), is received live at the display location (5) and the at least one surroundings property for the virtual reality is generated live at the display location (5) by means of at least one generating device (2) of the system (6),
wherein the generating device (2) comprises means (13, 14) emitting odorant to represent an odor as a first surroundings property, and wherein the representation of the virtual reality is controlled by a control unit (10) of the system (6)
**characterized in that**
the generating device (2) comprises as means (13, 14) a plurality of fans (13a, 13b, 13c), by means of which a wind direction is represented as a second surrounding property at the display location (5), so that a wind from a plurality of directions is represented,
the generating device (2) additionally comprises as means (13, 14) a plurality of nozzles (14a, 14b, 14c) for representing the odor as a first surrounding property, wherein an odorant is emitted by means of each nozzle (14a, 14b, 14c), and
**in that** at least two of the fans (13) are each assigned a nozzle (14) in order to achieve a directional dependence of the odor, so that the user (7) is presented with an odor coming from a specific direction.

2. The method of the preceding claim, **characterized in that** visual data from the recording location (4) for representing the visual virtual reality are received live at the display location (5) and are represented live at the display location (5) by means of the visual display unit (1).

3. The method of one of the preceding claims, **characterized in that** wind direction and fragrance and a temperature and/or atmospheric humidity are/is detected as a surroundings property at the recording location (4) and/or
that the at least first surrounding property and second surrounding property are/is detected by means of two surroundings sensors (8, 9, 3) at the recording location (4), and/or that wind direction and fragrance are detected at the display location (5), and temperature and/or atmospheric humidity are generated according to the surroundings properties detected at the recording location (4).

4. The method of one of the preceding claims, **characterized in that** the control unit (10) receives the at least first surroundings property and second surroundings property preferably by means of a data interface (11) and/or
the control unit (10) requests the at least first surroundings property and second surroundings property from the recording location (4) and/or
in particular by means of the control unit (10), the detection of the at least first surroundings property and second surroundings property at the recording location (4) and/or the detection of the visual data is controlled and/or instructed.

5. The method of one of the preceding claims, **characterized in that** the detection of the at least first surroundings property and second surroundings property at the recording location (4) is instructed out according to instructions from the display location (5).

6. The method of one of the preceding claims, **characterized in that** by means of the nozzles (14a, 14b, 14c) the odorant is dispensed, which generates the fragrance, and the odorant is dispensed in the area of the fan (13) and/or
the generating device (2) includes a fragrance device (15), in which the odorant is preferably mixed from multiple basic substances.

7. A system (6) for representing a virtual reality, wherein the system (6) is designed, in particular, such that it can be operated according to a method of one of the preceding claims,
including at least one visual display unit (1) for visually representing a virtual reality at a display location (5) and
including at least one generating device (2) for generating at least one surroundings property for the virtual reality at the display location (5), wherein the at least one surroundings property, which originates from a recording location (4), can be received live at the display location (5) and the at least one surroundings property can be generated live by means of the generating device (2), wherein the generating device (2) comprises means (13, 14) emitting odorant to represent a fragrance as a first surrounding property, and
including a control unit (10) by means of which the representation of the virtual reality is controlled,
**characterized in that**
the generating device (2) comprises a plurality of fans (13a, 13b, 13c) as means (13, 14), by means of which a wind direction is represented as a second surrounding property at the display location (5), so that a wind from several directions can be represented,
the generating device (2) additionally comprises a plurality of nozzles (14a, 14b, 14c) as means (13, 14) for representing the fragrance as a first surrounding property, wherein an odorant is emitted by means of each nozzle (14a, 14b, 14c), and
a nozzle (14) is assigned to at least two of the fans (13) in order to achieve a directional dependence of the fragrance, so that the user (7) can be presented with a fragrance coming from a specific direction..

8. The system of the preceding claim 7, **characterized in that** the control unit (10) can receive the at least first surroundings property and second surroundings property preferably live by means of a data interface (11) and/or
the control unit (10) can establish a connection to at least two surroundings sensors (8, 9, 3) at the receiving location (4), preferably by means of the data interface (11), in order to receive the at least first surroundings property live and/or ascertain the at least one surroundings property and second surroundings property live and/or to be able to determine the at least first surroundings property and second surroundings property..

9. The system of one of the preceding claims 7 or 8, **characterized in that** the control unit (10) can control the detection of the at least first surroundings property and second surroundings property at the recording location (4), in particular by means of the data interface (11), and/or
that the control unit (10) can request the at least first surroundings property and second surroundings property from the recording location (4), in particular by means of the data interface (11).

10. The system of one of the preceding claims 7 to 9, **characterized in that**, by means of the control unit (10), the at least first surroundings property and second surroundings property can be ascertained at the recording location (4) by at least two surroundings sensor (8, 9, 3).

11. The system of one of the preceding claims 7 to 10, **characterized in that** the generating device (2) comprises a fragrance device (15) for generating fragrances for a virtual reality, wherein the fragrance device (15) comprises multiple containers (16) for storing basic substances, wherein the fragrance device (15) can mix the fragrances by means of the basic substances, and/or
the fragrance device (15) includes multiple, in particular exchangeable, containers (16).

12. The system of the preceding claim 7, **characterized in that** the generating device (2) comprises a fragrance device (15) for generating fragrances for a virtual reality, wherein the fragrance device (15) comprises at least one container (16) for storing a fragrance and/or base substances..

13. The system of the preceding claim, **characterized in that** the fragrance device (15) comprises multiple containers (16) for storing basic substances and/or odorants, and/or
the containers (16) are exchangeable.

## Revendications

1. Procédé de représentation d'une réalité virtuelle, pour un système (6) de représentation de la réalité virtuelle,
dans lequel une réalité virtuelle est représentée visuellement à un lieu d'affichage (5) au moyen d'au moins une unité d'affichage visuelle (1) du système (6),
dans lequel au moins une propriété environnementale provenant d'un lieu d'admission (4) est reçue en direct au lieu d'affichage (5) et
dans lequel l'au moins une propriété environnementale pour la réalité virtuelle est générée en direct au lieu d'affichage (5) au moyen d'au moins un dispositif générateur (2) du système (6), le dispositif générateur (2) comprenant des moyens (13, 14) qui émettent une substance odorante pour représenter une odeur en tant que première propriété environnementale, et
la représentation de la réalité virtuelle étant contrôlée au moyen d'une unité de contrôle (10) du système (6),
**caractérisé en ce que**
le dispositif générateur (2) comprend en tant que moyens (13, 14) plusieurs ventilateurs (13a, 13b, 13c) au moyen desquels une direction du vent est représentée en tant que deuxième propriété environnementale au lieu d'affichage (5), de sorte qu'un vent est représenté depuis plusieurs directions,
le dispositif générateur (2) comprend en outre en tant que moyens (13, 14) plusieurs buses (14a, 14b, 14c) pour représenter l'odeur en tant que première propriété environnementale, une substance odorante étant émise au moyen de chaque buse (14a, 14b, 14c), et
au moins deux des ventilateurs (13) sont associés chacun à une buse (14) pour obtenir une dépendance de direction de l'odeur, de sorte qu'il est représenté à l'utilisateur (7) qu'une odeur provient d'une direction déterminée.

2. Procédé selon la revendication précédente, **caractérisé en ce que** des données visuelles sont reçues en direct depuis le lieu d'admission (4) pour la représentation de la réalité virtuelle visuelle au lieu d'affichage (5) et sont représentées en direct au lieu d'affichage (5) au moyen de l'unité d'affichage visuelle (1).

3. Procédé selon l'une des revendications précédentes, **caractérisé en ce que** la direction du vent et l'odeur sont détectées en tant que propriété environnementale au lieu d'admission (4), et une température et/ou une humidité de l'air sont détectées, et/ou
l'au moins première propriété environnementale et la deuxième propriété environnementale sont détectées au moyen de deux capteurs environnementaux (8, 9, 3) au lieu d'admission (4), et/ou la direction du vent et l'odeur sont détectées au lieu d'affichage (5), et la température et/ou l'humidité de l'air sont générées en fonction des propriétés environnementales détectées au lieu d'admission (4).

4. Procédé selon l'une des revendications précédentes, **caractérisé en ce que** l'unité de contrôle (10) reçoit l'au moins première propriété environnementale et la deuxième propriété environnementale de préférence au moyen d'une interface de données (11), et/ou
l'unité de contrôle (10) demande l'au moins première propriété environnementale et la deuxième propriété environnementale au lieu d'admission (4) et/ou
la détection de l'au moins première propriété environnementale et de la deuxième propriété environnementale au lieu d'admission (4) et/ou la détection des données visuelles sont contrôlées et/ou ordonnées, en particulier au moyen de l'unité de contrôle (10).

5. Procédé selon l'une des revendications précédentes, **caractérisé en ce que** la détection de l'au moins première propriété environnementale et de la deuxième propriété environnementale au lieu d'admission (4) est ordonnée au moyen d'instructions du lieu d'affichage (5).

6. Procédé selon l'une des revendications précédentes, **caractérisé en ce que** la substance odorante qui génère l'odeur est émise au moyen des buses (14a, 14b, 14c) et **en ce que** la substance odorante est émise dans la zone du ventilateur (13) et/ou
le dispositif générateur (2) comprend un dispositif d'odeur (15) dans lequel la substance odorante est mélangée de préférence à partir de plusieurs substances de base.

7. Système (6) de représentation d'une réalité virtuelle, dans lequel le système (6) est conçu en particulier de manière à pouvoir fonctionner selon un procédé selon l'une des revendications précédentes, avec au moins une unité d'affichage visuelle (1) pour la représentation visuelle d'une réalité virtuelle à un lieu d'affichage (5) et
avec au moins un dispositif générateur (2) pour la génération en direct d'au moins une propriété environnementale pour la réalité virtuelle au lieu d'affichage (5),
dans lequel l'au moins une propriété environnementale provenant d'un lieu d'admission (4) est reçue en direct au lieu d'affichage (5) et l'au moins une propriété environnementale est générée en direct au moyen de l'au moins un dispositif générateur (2), le dispositif générateur (2) comprenant des moyens (13, 14) qui émettent une substance odorante pour représenter une odeur en tant que première propriété environnementale, et
avec une unité de contrôle (10) au moyen de laquelle la représentation de la réalité virtuelle est contrôlée,
**caractérisée en ce que** le dispositif générateur (2) comprend en tant que moyens (13, 14) plusieurs ventilateurs (13a, 13b, 13c) au moyen desquels une direction du vent est représentée en tant que deuxième propriété environnementale au lieu d'affichage (5), de sorte qu'un vent peut être représenté depuis plusieurs directions,
le dispositif générateur (2) comprend en outre en tant que moyens (13, 14) plusieurs buses (14a, 14b, 14c) pour représenter l'odeur en tant que première propriété environnementale, une substance odorante étant émise au moyen de chaque buse (14a, 14b, 14c), et
au moins deux des ventilateurs (13) sont associés chacun à une buse (14) pour obtenir une dépendance de direction de l'odeur, de sorte qu'il peut être représenté à l'utilisateur (7) qu'une odeur provient d'une direction déterminée.

8. Système selon la revendication précédente 7, **caractérisé en ce que** l'unité de contrôle (10) peut recevoir l'au moins première propriété environnementale et la deuxième propriété environnementale de préférence en direct au moyen d'une interface de données (11) et/ou l'unité de contrôle (10) peut former de préférence au moyen de l'interface de données (11) une connexion à au moins deux capteurs environnementaux (8, 9, 3) au lieu d'admission (4) pour recevoir l'au moins première propriété environnementale et la deuxième propriété environnementale en direct et/ou pour pouvoir déterminer l'au moins première propriété environnementale et la deuxième propriété environnementale.

9. Système selon l'une des revendications précédentes 7 ou 8, **caractérisé en ce que** l'unité de contrôle (10) peut contrôler la détection de l'au moins première propriété environnementale et de la deuxième propriété environnementale au lieu d'admission (4), en particulier au moyen de l'interface de données (11) et/ou
l'au moins première propriété environnementale et la deuxième propriété environnementale peuvent être demandées au lieu d'admission (4), en particulier au moyen de l'interface de données (11).

10. Système selon l'une des revendications précédentes 7 à 9, **caractérisé en ce que** l'au moins première propriété environnementale et la deuxième propriété environnementale peuvent être déterminées au moyen de l'unité de contrôle (10) par au moins deux capteurs environnementaux (8, 9, 3) au lieu d'admission (4).

11. Système selon l'une des revendications précédentes 7 à 10, **caractérisé en ce que** le dispositif générateur (2) comprend un dispositif d'odeur (15) pour la génération d'odeurs pour une réalité virtuelle, le dispositif d'odeur (15) comprenant plusieurs réservoirs (16) pour le stockage de substances de base, le dispositif d'odeur (15) pouvant mélanger les odeurs au moyen des substances de base et/ou le dispositif d'odeur (15) comprend plusieurs réservoirs (16), en particulier remplaçables.

12. Système selon la revendication précédente 7,
**caractérisé en ce que** le dispositif générateur (2) comprend un dispositif d'odeur (15) pour la génération d'odeurs pour une réalité virtuelle, le dispositif d'odeur (15) comprenant
au moins un réservoir (16) pour le stockage d'une substance odorante et/ou de substances de base.

13. Système selon la revendication précédente, **caractérisé en ce que** le dispositif d'odeur (15) comprend plusieurs réservoirs (16) pour le stockage de substances de base et/ou de substances odorantes et/ou les réservoirs (16) sont remplaçables.
